(19) Europäisches Patentamt
European Patent Office
Office européen des brevets

(11) **EP 2 629 082 B1**

(12) **EUROPÄISCHE PATENTSCHRIFT**

(45) Veröffentlichungstag und Bekanntmachung des Hinweises auf die Patenterteilung:
**01.11.2017 Patentblatt 2017/44**

(51) Int Cl.:
***G01N 21/35*** *(2014.01)*

(21) Anmeldenummer: **13155419.8**

(22) Anmeldetag: **15.02.2013**

(54) **Vorrichtung zur Detektion eines Partialdrucks und Verfahren zum Betreiben derselben**

Device for detecting a partial pressure and method for operating the same

Dispositif de détection d'une pression partielle et procédé de fonctionnement de celui-ci

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.02.2012 DE 102012101313**

(43) Veröffentlichungstag der Anmeldung:
**21.08.2013 Patentblatt 2013/34**

(73) Patentinhaber: **Kongsberg Maritime Contros GmbH**
**24148 Kiel (DE)**

(72) Erfinder: **Fietzek, Peer**
**24148 Kiel (DE)**

(74) Vertreter: **Knoop, Philipp**
**VKK Patentanwälte**
**An der Alster 84**
**20099 Hamburg (DE)**

(56) Entgegenhaltungen:
**EP-A1- 0 900 365          EP-A2- 0 551 924**
**WO-A1-98/40722          DE-A1-102006 035 788**
**DE-B3-102006 019 705   US-A1- 2007 261 475**

EP 2 629 082 B1

**Beschreibung**

Die vorliegende Erfindung betrifft eine Vorrichtung zur Detektion eines Partialdrucks eines in einem Umgebungsgas enthaltenen Messgases, gemäß dem Oberbegriff des Patentanspruchs 1.

Die vorliegende Erfindung betrifft außerdem ein Verfahren zum Betreiben einer einen Messraum und einen auf das Messgas empfindlichen Messgassensor zum Messen einer für den Partialdruck des Messgases im Messraum kennzeichnenden Größe umfassenden Vorrichtung, zur Detektion eines Partialdrucks eines in einem Umgebungsgas enthaltenen Messgases.

Eine Vorrichtung der eingangs genannten Art sowie ein Verfahren zum Betreiben derselben sind beispielsweise aus der DE 10 2006 019 705 B3 bekannt. Diese betrifft ein dynamisches Messwertfilter für eine Gassensoranordung sowie ein Verfahren zur Verarbeitung von zeitdiskreten Messwerten. Mit dem bekannten Verfahren wird ohne Veränderung der konstruktiven Gegebenheiten das Ansprechverhalten der Gassensoranordung verbessert, indem ein Filterschritt durchgeführt wird. Eine Kompensation des Ansprechverhaltens ist nicht vorgesehen. Die WO 98/40722 A1 offenbart die Spülung eines Gassensors, um sicherzustellen, dass das Messergebnis nicht durch in der Messkammer vorhandenes Restgas verfälscht wird. Eine Kompensation des Ansprechverhaltens des Gassensors ist auch hier nicht vorgesehen. Aus der DE 10 2006 035 788 A1 ist ebenfalls eine Vorrichtung zur Detektion eines Partialdrucks bekannt. Derartige Vorrichtungen werden unter anderem verwendet, um eine messtechnische Erfassung von Gasen durchzuführen. Bei geeigneter Konfiguration, insbesondere mit einer den Messraum vom Umgebungsgas trennenden Membran, können mit derartigen Vorrichtungen insbesondere Gasmessungen unter Wasser durchgeführt werden. Insbesondere die Leckagedetektion an Unterwasserpipelines oder anderen Unterwasserstrukturen sowie allgemein die Lokalisierung von künstlichen und natürlichen Gasaustritten am Meeresboden zu technischen oder wissenschaftlichen Zwecken gehört zu Anwendungsgebieten der gattungsgemäßen Detektionsvorrichtung.

Bei derartigen Anwendungen ist es in vielen Fällen erforderlich, Messungen bei Bedingungen durchzuführen, bei denen der Partialdruck des in dem Umgebungsgas enthaltenen Messgases, welches von der bekannten Vorrichtung zu erfassen ist, zeitlich variabel ist. Dies kann zum einen daran liegen, dass bei stationärer Anordnung der Detektionsvorrichtung eine zeitliche Änderung des Partialdrucks des Messgases auftritt. Zum anderen kann dies der Fall sein, wenn ein im Wesentlichen stationäres Partialdruckgefüge des Messgases erfasst wird, indem die erfindungsgemäße Detektionsvorrichtung durch ein Messgebiet mit räumlich unterschiedlichen Partialdrücken des Messgases hindurchbewegt wird. In derartigen Fällen ist es für eine optimale Erfassung des Partialdrucks des in dem Umgebungsgas enthaltenen Messgases wichtig, dass eine räumliche bzw. zeitliche Zuordnung von Messsignalen des Messgassensors zu den tatsächlichen Bedingungen des Umgebungsgases möglich ist Es hat sich jedoch gezeigt, dass bei den bekannten Vorrichtungen eine hinreichende zeitliche bzw. räumliche Zuordnung in manchen Fällen nicht möglich ist.

Aufgabe der vorliegenden Erfindung ist es daher, eine Vorrichtung sowie ein Verfahren der eingangs genannten Art anzugeben, welche eine messtechnische Erfassung des Partialdrucks eines in einem Umgebungsgas enthaltenen Messgases mit verbesserter zeitlicher bzw. räumlicher Auflösung ermöglichen.

Diese Aufgabe wird erfindungsgemäß mit einer gattungsgemäßen Vorrichtung gelöst, bei welcher Mittel zur Bestimmung einer Ansprechcharakteristik der Vorrichtung gemäß den Merkmalen des Anspruchs 1 vorgesehen sind.

Erfindungsgemäß umfassen die Mittel zur Bestimmung einer Ansprechcharakteristik Mittel zum Durchströmen des Messraumes mit einem Nullgas, auf welches der Messgassensor und/oder der Korrekturgassensor nicht empfindlich ist. Diese Maßnahme ermöglicht es mit Vorteil, ein sogenanntes Zeroing des Messgassensors nach Bedarf *in situ* durchzuführen. Bei dem für sich genommen bekannten Zeroing-Verfahren werden im Detektor Bedingungen geschaffen, die zu einer Nullpunktsmessung des Detektors führen, indem dem Detektor ein Gas oder Gasgemisch zugeführt wird, auf welches er nicht anspricht. Ausgehend von diesem definierten Nullzustand lässt sich durch geeignete Verfahren das Ansprechverhalten der Messvorrichtung messtechnisch erfassen und charakterisieren. Dies ermöglicht wiederum eine rechnerische Kompensation des Ansprechverhaltens aus den im normalen Messbetrieb gewonnenen Messdaten. Gegenüber einem herkömmlichen Zeroing der Vorrichtung ex situ, beispielsweise im Labor, ergeben sich vielfältige Vorteile. Insbesondere ist bei einem *in situ* Zeroing sichergestellt, dass eine etwaige Abhängigkeit des Ansprechverhaltens von Umgebungsparametern, wie z.B. Temperatur oder Druck, berücksichtigt ist. Diese Form des Zeroing eignet sich insbesondere für Fälle, in denen beim Zeroing ausreichend Messgas im Umgebungsgas enthalten ist.

Erfindungsgemäß ist eine Steuer- und Auswerteeinheit zur Steuerung der Mittel zum Durchströmen des Messraumes vorgesehen, um ein Durchströmen des Messraumes mit einem Nullgas gezielt ein- und auszuleiten. Anhand der Steuer- und Auswerteeinheit kann ein Zeroing mit einem Nullgas somit mit Vorteil *in situ* vorgenommen werden. Insbesondere kann bei Verdacht auf veränderte Umgebungsbedingungen ein Zeroing *in situ* wiederholt werden, um eine veränderte Ansprechcharakteristik aufzuzeichnen.

Erfindungsgemäß ist die Steuer- und Auswerteeinheit zur rechnerischen Bestimmung eines Ansprechverhaltens aus einer zeitlichen Reihe über die Auslesemittel aufgezeichneter Messdaten ausgebildet. Dies ermöglicht es erfindungsgemäß, anhand der Steuer- und Auswerteeinheit die Mittel zum Durchströmen des Messraumes mit einem Nullgas zu aktivieren, um einen Nullzustand zu erzeugen. Anschließend kann anhand der Steuer- und Auswerteeinheit eine Sequenz von Messdaten aufgezeichnet werden, sobald die Mittel zum Durchströmen des Messraumes mit einem Nullgas wieder deaktiviert sind und wieder Umgebungsgas in die erfindungsgemäße Detektionsvorrichtung eintritt. Die Ansprechcharakteristik kann unter Zugrundelegung eines geeigneten mathematischen Modells oder auf andere Weise bestimmt werden. Mit Vorteil umfasst das auf diese Weise bestimmte dynamische Ansprechverhalten sämtliche das Ansprechen des Messgassensors verzögernde Effekte. Dazu zählen neben einer Dynamik des Messgassensors selber insbesondere auch Verzögerungen, die das Umgebungsgas beim Eintritt in den Messraum erleidet. Dies ist insbesondere bei der Verwendung einer Membran, über welche das Umgebungsgas in den Messraum eintritt, von Bedeutung.

Die Erfindung sieht vor, dass die Steuer- und Auswerteeinheit zur rechnerischen Kompensation eines Ansprechverhaltens der über die Auslesemittel aufgezeichneten Messdaten ausgestaltet ist. Auf diese Weise ist es möglich, eine zeitlich bzw. räumlich zutreffende Zuordnung von Messdaten zu tatsächlich räumlichen bzw. zeitlichen Änderungen des Partialdrucks des Messgases zu bestimmen. Als für die rechnerische Kompensation zugrundezulegendes zeitliches Ansprechverhalten kann einerseits eine in der Auswerte- und Auswerteeinheit zuvor abgelegte Ansprechcharakteristik herangezogen werden. Andererseits kann ein *in situ* bestimmtes zeitliches Ansprechverhalten für die rechnerische Kompensation verwendet werden.

In Ausgestaltung der Erfindung können die Mittel zur Bestimmung einer Ansprechcharakteristik einen auf ein Korrekturgas empfindlichen Korrekturgassensor zum Messen einer für einen Partialdruck des Korrekturgases im Messraum kennzeichnenden Messgröße sowie Auslesemittel zum Auslesen des Korrekturgassensors umfassen, wobei vorzugsweise das Korrekturgas in dem Umgebungsgas enthalten und/oder von dem Messgas verschieden ist. Durch diese Maßnahme kann ein Zeroing durchgeführt werden, ohne dass im Umgebungsgas Messgas mit einem Partialdruck oberhalb einer Detektionsschwelle vorliegen muss.

Gemäß einer bevorzugten Ausführungsform der erfindungsgemäßen Vorrichtung weist sie eine Nullgasquelle zur Bereitstellung des Nullgases auf. Die Nullgasquelle kann im einfachsten Fall eine Patrone oder ähnliches sein, welche mit dem Nullgas gefüllt ist. Die Mittel zum Durchströmen des Messraums mit dem Nullgas würden gemäß dieser Ausführungsform eine Durchströmung des Messraumes mit dem Inhalt dieser Patrone einerseits und ein Abriegeln des Messraumes gegenüber dem Umgebungsgas andererseits bewirken.

Gemäß einer besonders günstigen Ausgestaltungsform ist die Nullgasquelle zur Erzeugung des Nullgases aus dem Umgebungsgas ausgebildet. Mit Vorteil entfällt gemäß dieser Ausführungsform die Notwendigkeit, einen Vorrat an Nullgas vorzusehen.

Insbesondere kann die Nullgasquelle Mittel zum Entfernen des Messgases aus dem Umgebungsgas umfassen, um das Nullgas aus dem Umgebungsgas zu erzeugen. -Beispielsweise kann das Umgebungsgas in der Nullgasquelle zurückgehalten werden, indem es dort chemisch gebunden wird. So kann beispielsweise im Falle eines auf $CO_2$ empfindlichen Messgassensors die Nullgasquelle eine Säule aus einer $CO_2$ absorbierenden Chemikalie, wie z.B. Natronkalk oder Ascarit, umfassen. Die Entfernung des $CO_2$ aus dem Umgebungsgas erfolgt in diesem Falle in für sich genommen bekannter Weise durch Umwandlung des $CO_2$ in $CaCO_3$. Zum Binden von Kohlenwasserstoffen kann auch Aktivkohle eingesetzt werden, um aus dem Umgebungsgas ein Nullgas zu erzeugen, wenn der Messgassensor für Kohlenwasserstoffe empfindlich ist. Erfindungsgemäß kann die Nullgasquelle gleichermaßen Mittel zum chemischen Umwandeln des Messgases derart umfassen, dass der Messgassensor und/oder der Korrekturgassensor auf bei der Umwandlung entstehende gasförmige Umwandlungsprodukte nicht empfindlich ist. Beispielsweise kann eine Oxidation des Messgases vorgenommen werden, vorzugsweise unter Verwendung des im Umgebungsgas vorhandenen Sauerstoffanteils. Es kann im Rahmen der Erfindung auch Sauerstoff aus einem Reservoir oder einer Sauerstoffquelle verwendet werden. Insbesondere kann im Falle eines auf CH4 empfindlichen Messgassensors das Methan mittels eines Pellistors oder eines thermokatalytischen Elements zu Kohlendioxid oxidiert werden. Im Falle eines für Wasserdampf empfindlichen Messgassensors kann die chemische Umwandlung mittels Magnesiumperchlorat ((Hydrat), $Mg (ClO_4)_2$, mittels Kalziumsulfat ($CaSO_4$) oder mittels Kiesel- oder Silikagel ($SiO_2$) durchgeführt werden.

Da das Umgebungsgas, insbesondere bei Durchführung der Messung unter Wasser, in der Praxis stets einen Feuchteanteil, also einen Wasseranteil, aufweist, kann das Zeroing auch dadurch bewirkt werden, dass der Wasseranteil aus dem Umgebungsgas zeitweise entfernt wird. Man verwendet dann also als Korrekturgassensor einen Sensor, der auf den im Umgebungsgas enthaltenen Wasseranteil empfindlich ist, sodass der im Umgebungsgas enthaltene gasförmige Wasseranteil als Korrekturgas fungiert. Für diesen Korrekturgassensor ist trockenes Umgebungsgas ein Nullgas. Als Nullgasquelle kann man in diesem Falle jedes geeignete Mittel verwenden, um das Umgebungsgas zu trocken, diesem also den Wasseranteil zu entziehen. Dazu eignet sich beispielsweise Silikagel.

Um die erfindungsgemäße Vorrichtung unter Wasser einsetzen zu können, ist in spezieller Ausgestaltung der Erfindung stromaufwärts von der Einlassöffnung und/oder stromabwärts von der Auslassöffnung eine für das Umgebungsgas durchlässige Membran derart angeordnet, dass das Umgebungsgas durch die Membran in den Messraum gelangt und/oder das Umgebungsgas die Vorrichtung durch die Membran verlässt.

Insbesondere bei der Anordnung einer Membran im Gaseinlass ist es erfindungsgemäß vorteilhaft, das dynamische Ansprechverhalten *in situ* jeweils für gegebene Umgebungsparameter durchführen zu können. Denn das zeitliche Ansprechverhalten wird in diesem Fall auch durch Eigenschaften der Membran beeinflusst.

Um ein *in situ* Zeroing besonders gut durchführen zu können, kann eine die Einlassöffnung mit der Auslassöffnung verbindende Bypass-Leitung vorgesehen sein. Insbesondere in Fällen, in denen das Nullgas aus dem Umgebungsgas erzeugt wird, kann mittels der Bypass-Leitung das in dem Messraum befindliche Umgebungsgas statt zurück in die Umgebung abgegeben zu werden, durch die Nullgasquelle geleitet werden, um so aus dem Umgebungsgas das Nullgas zu erzeugen.

Insbesondere kann der Messgassensor und/oder der Korrekturgassensor erfindungsgemäß einen optischen Messgassensor, vorzugsweise einen nicht-dispersiven Infrarotsensor und/oder ein auf einer durchstimmbaren Laserdiode basierendes Spektrometer und/oder einen auf einer durchstimmbaren Laserdiode basierenden Sensor, umfassen und/oder es kann eine Pumpe vorgesehen sein, um Umgebungsgas durch den Messraum zu zirkulieren. Ein optischer Messgassensor ist im Zusammenspiel mit einer Druck- und/oder Temperaturmessung des in den Messraum eintretenden Umgebungsgases geeignet, den Partialdruck des Messgases zu bestimmen.

Im Falle einer Vorrichtung für den Einsatz in Flüssigkeiten, wo das Umgebungsgas in der Flüssigkeit gelöst ist und durch eine Membran in den Messraum eintritt, kann eine Pumpe vorgesehen sein, um die Membran von außen mit gelöstes Umgebungsgas enthaltender Flüssigkeit gleichmäßig anzuströmen und so den Äqulibrationsprozesss zu beschleunigen.

Hinsichtlich des Verfahrens wird die der Erfindung zugrundeliegende Aufgabe mit einem Verfahren der eingangs genannten Art gelöst, welches ein Verfahren zur Bestimmung einer Ansprechcharakteristik des Messgassensors umfasst, bei welchem

(a) der Messraum mit einem Nullgas, auf welches der Messgassensor und/oder ein auf ein Korrekturgas empfindlicher Korrekturgassensor (51) nicht empfindlich ist, durchströmt wird, wobei gleichzeitig ein Ausgangssignal des Messgassensors und/oder ein Ausgangssignal des Korrekturgassensors (51) beobachtet wird, bis dieses einen im wesentlichen gleichbleibenden Minimalwert erreicht;

(b) der Messraum mit Umgebungsgas durchströmt wird, nachdem das Ausgangssignal einen im wesentlichen gleichbleibenden Minimalwert erreicht hat; wobei mit Beginn der Durchströmung mit Umgebungsgas das Ausgangssignal des Messgassensors und/oder das Ausgangssignal des Korrekturgassensors (51) während eines Kalibrationszeitintervalls zeitaufgelöst in einer Messreihe aufgezeichnet wird;

(c) anhand einer Auswertung der Messreihe die Ansprechcharakteristik bestimmt wird,

wobei zumindest die Schritte (a) und (b) *in situ* durchgeführt werden.

Um ein Zeroing auch durchführen zu können, wenn im Umgebungsgas kein oder ein für eine Detektion zu geringer Anteil an Messgas enthalten ist, kann in Weiterbildung der Erfindung zur Auswertung der Messreihe zunächst eine Korrekturansprechcharakteristik auf Basis des Ausgangssignals des Korrekturgassensors bestimmt werden und anschließend aus der Korrekturansprechcharakteristik die Ansprechcharakteristik der Vorrichtung bestimmt werden. Für die Bestimmung der Ansprechcharakteristik der Vorrichtung ausgehend von der Korrekturansprechcharakteristik kann erfindungsgemäß auf beispielsweise durch Laborversuche erlangte Erkenntnisse zurückgegriffen werden.

Das erfindungsgemäße Verfahren ermöglicht mit Vorteil die Bestimmung eines Ansprechverhaltens der Vorrichtung *in situ,* das heißt direkt in der Umgebung, in welcher die Vorrichtung zur Detektion eines Partialdrucks eines in dem Umgebungsgas enthaltenen Messgases eingesetzt wird. Da die Ansprechcharakteristik möglicherweise von Umgebungsparametern, wie insbesondere Temperatur oder Druck, ist es anhand des erfindungsgemäßen Verfahrens mit Vorteil möglich, die Ansprechcharakteristik des gesamten Messsystems der Vorrichtung unter Berücksichtigung der jeweiligen Umgebungsbedingungen zu bestimmen. Dies wiederum ermöglicht es mit Vorteil, eine bessere zeitliche bzw. räumliche Zuordnung von Messwerten zu der tatsächlichen Messgröße zu erhalten. Dadurch, dass die Bestimmung der Ansprechcharakteristik einerseits *in situ* erfolgt und dass andererseits die Ansprechcharakteristik der gesamten Messvorrichtung und nicht lediglich beispielsweise des isolierten Messgassensor erfolgt, ist zudem sichergestellt, dass sämtliche die Ansprechcharakteristik beeinflussende Größen, wie beispielsweise auch Eigenschaften einer im Einlass- und Auslassströmungspfad enthaltenen Membran berücksichtigt werden. Daher ermöglicht das erfindungsgemäße Verfahren mit Vorteil auch die Erfassung von die Messung beeinflussenden Veränderungen der Membran, wie zum Beispiel Änderungen der Membrandicke, der Konditionierung, der Anströmung oder des Zustands mit Blick auf einen Bewuchs oder eine Verstopfung. Das erfindungsgemäße Verfahren ermöglicht zudem eine Nullpunktskalibration das Messgassensors bei Erreichen des im wesentlichen gleichbleibenden Minimalwerts, da an diesem bekannt ist, dass kein Messgas detektiert wird.

In Ausgestaltung des erfindungsgemäßen Verfahrens ist vorgesehen, dass das Ausgangssignal des Messgassensors anhand der Ansprechcharakteristik korrigiert wird, um eine zeitliche Zuordnung des Ausgangssignals zu dem Partialdruck des Messgases zum Zeitpunkt der Aufnahme des Ausgangssignals zu erhalten. Das erfindungsgemäße Verfahren ermöglicht auf diese Weise eine um die Ansprechcharakteristik korrigierte Erfassung des Partialdrucks eines in einem Umgebungsgas enthaltenen Messgases. Dies ist insbesondere von Vorteil, wenn räumliche oder zeitliche Veränderungen des Partialdrucks des Messgases zu erwarten sind, etwa weil die Detektionsvorrichtung durch ein Messgebiet hindurchbewegt wird.

Wenn in weiterer vorteilhafter Ausgestaltung des erfindungsgemäßen Verfahrens das Kalibrationszeitintervall so gewählt wird, dass der Partialdruck des Messgases und/oder jener des Korrekturgases während des Kalibrationszeitintervalls im Wesentlichen konstant ist, ist eine besonders präzise Bestimmung der Ansprechcharakteristik möglich. Denn bei dem Verfahren zur Bestimmung der Ansprechcharakteristik liegen definierte Eingangs- und Ausgangszustände vor, welche nicht veränderlich sind, so dass während des Kalibrationszeitintervalls die Ansprechcharakteristik als Sprungantwort bestimmt werden kann.

Das erfindungsgemäße Verfahren wird noch verbessert, wenn für die Bestimmung der Ansprechcharakteristik ein erster Abschnitt der Messreihe, welcher während einer Durchmischungsphase des Umgebungsgases mit dem Nullgas in dem Messraum aufgezeichnet wurde, nicht berücksichtigt wird. Es hat sich experimentell gezeigt, dass die Ansprechcharakteristik, welches innerhalb des Kalibrationsintervalls messbar ist, in einem ersten Abschnitt der Messreihe mit einem anderen mathematischen Modell beschreibbar ist als die Ansprechcharakteristik in einem zweiten, sich an den ersten Abschnitt anschließenden Abschnitt der Messreihe. Die Erfinder vermuten, dass das Verhalten in dem ersten Abschnitt der Messreihe darauf zurückzuführen ist, dass das Nullgas zunächst durch das Umgebungsgas in dem Messraum ersetzt werden muss. Da diese Durchmischungsphase mit dem Zeroing-Verfahren zusammenhängt und im normalen Messbetrieb entfällt, hat sich empirisch gezeigt, dass zur Korrektur von Messwerten im normalen Messbetrieb anhand der zuvor bestimmten Ansprechcharakteristik eine Ansprechcharakteristik am besten geeignet ist, bei welcher der besagte erste Abschnitt der Messreihe außer Betracht bleibt.

Insbesondere kann in bevorzugter Ausgestaltung des erfindungsgemäßen Verfahrens der erste Abschnitt der Messreihe sich bis zu einem Übergangspunkt von einem im Wesentlichen linearen zu einem im Wesentlichen exponentiellen Verlauf erstrecken. Die Bestimmung dieses Übergangspunkts kann erfindungsgemäß erfolgen, indem zunächst eine lineare Regression der Anfangswerte der Messreihe vorgenommen wird und im zweiten Schritt das Residuum zwischen den Messwerten und der Regressionsgeraden ermittelt wird. Sobald dieses Residuum einen vorbestimmten Schwellwert übersteigt, ist der Übergangspunkt erreicht. Zur Bestimmung des Übergangspunktes kann erfindungsgemäß gleichermaßen eine Auswertung der zeitlichen Änderungsrate der Messwerte durchgeführt werden, wobei der Übergangspunkt durch eine signifikante Reduzierung der Änderungsrate gekennzeichnet ist.

In bevorzugter Ausgestaltung des erfindungsgemäßen Verfahrens wird die Ansprechcharakteristik anhand einer Regressionsanalyse der Messreihe bestimmt, wobei vorzugsweise folgendes Modell angesetzt wird:

$$p_{(t)} = p_{water} + (p_{(t_0)} - p_{water})e^{-\frac{t}{\tau}},$$

wobei p(t) das Ausgangssignal zum Zeitpunkt t, $p_{water}$ den Partialdruck des Messgases innerhalb des Umgebungsgases, $p(t_0)$ das Ausgangssignal zum Zeitpunkt $t_0$ und $\tau$ eine Zeitkonstante bezeichnen. Es hat sich gezeigt, dass eine Korrektur von Messwerten anhand der mit der genannten Formel bestimmten Ansprechcharakteristik, charakterisiert durch den Parameter $\tau$, besonders geeignet ist, den realen Wert der Messgröße zu erfassen.

In bevorzugter Ausgestaltung des erfindungsgemäßen Verfahrens wird das Ausgangssignal des Messgassensors anhand der Ansprechcharakteristik korrigiert, indem das Ausgangssignal p(t) zum Zeitpunkt t dem Partialdruck p water des Messgases innerhalb des Umgebungsgases zum Zeitpunkt der Aufnahme des Ausgangssignals gemäß folgender Formel zugeordnet wird:

$$p_{water} = \frac{p_{(t)} - p_{(t_0)}e^{-\frac{t}{\tau}}}{1 - e^{-\frac{t}{\tau}}},$$

wobei $p(t_0)$ das Ausgangssignal zum Zeitpunkt $t_0$ bezeichnet.

Eine iterative Korrektur des Ausgangssignals des Messgassensors anhand der Ansprechcharakteristik kann in diesem Sinne insbesondere mit folgender Formel erhalten werden:

$$p_{corrected,t_{i+1}} = \frac{p_{(t_{i+1})} - p_{(t_i)}e^{-\frac{\Delta t}{\tau}}}{1 - e^{-\frac{\Delta t}{\tau}}} \quad \text{mit} \quad \Delta t = t_{i+1} - t_i$$

Das Intervall ΔT, also die Abtastrate, muss mindestens so klein gewählt werden, dass der Partialdruck des Messgases innerhalb des Intervalls ΔT als konstant angenommen werden kann. Es richtet sich somit nach dem Partialdruckgradienten des Messgases, welcher bei der jeweiligen Messung zu erwarten ist. Andererseits richtet sich die Abtastrate auch nach der Geschwindigkeit, mit welcher die Detektionsvorrichtung durch das Messmedium bewegt wird.

In Weiterbildung des erfindungsgemäßen Verfahrens ist vorgesehen, dass das Verfahren zur Bestimmung einer Ansprechcharakteristik mehrmals, insbesondere nach jeder signifikanten Änderung eines oder mehrerer Umgebungsparameter, durchgeführt wird. Wenn beispielsweise eine Temperaturänderung oder Druckänderung festgestellt wird, wird gemäß dieser Ausgestaltungsvariante der Erfindung eine erneute Bestimmung der Ansprechcharakteristik vorgenommen.

Die Erfindung wird in einer bevorzugten Ausführungsform unter Bezugnahme auf eine Zeichnung beispielhaft beschrieben, wobei weitere vorteilhafte Einzelheiten den Figuren der Zeichnung zu entnehmen sind.

Funktionsmäßig gleiche Teile sind dabei mit denselben Bezugzeichen versehen.

Die Figuren der Zeichnung zeigen im Einzelnen:

Figur 1: schematische Darstellung einer bevorzugten Ausführungsform für den Unterwassereinsatz einer erfindungsgemäßen Detektionsvorrichtung;

Figur 2: schematische Darstellung zur Veranschaulichung einer bevorzugten Ausgestaltungsform des erfindungsgemäßen Verfahrens zur Bestimmung einer Ansprechcharakteristik;

Figur 3: beispielhafte Messreihe, welche bei der Durchführung des Verfahrens zur Bestimmung der Ansprechcharakteristik ermittelt wurde, zwecks Veranschaulichung einer bevorzugten Ausgestaltung des Verfahrens zur Bestimmung der Ansprechcharakteristik;

Figur 4: schematische Darstellung einer anderen bevorzugten Ausführungsform für den Unterwassereinsatz einer erfindungsgemäßen Detektionsvorrichtung.

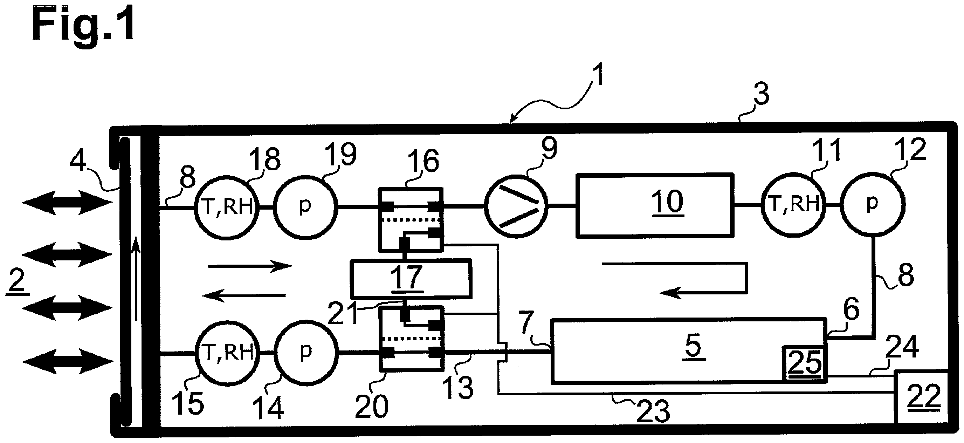

Die Figur 1 zeigt in einer schematischen Schnittansicht einen Partialdrucksensor 1, welcher in Wasser 2 eingetaucht ist. Der Partialdrucksensor 1 weist ein wasserdichtes Gehäuse 3 auf. Das wasserdichte Gehäuse 3 ist an einer Seite zu dem umgebenden Wasser 2 über einen Membranequilibrator 4 geöffnet. Der Membranequilibrator 4 ist für in dem umgebenden Wasser 2 enthaltenes Umgebungsgas durchlässig, nicht aber für Wasser. Im durch das Gehäuse 3 bzw. den Membranequilibrator 4 von dem umgebenden Wasser 2 abgegrenzten Innern des Partialdrucksensors 1 ist als Kernstück ein Messgassensor 5 angeordnet. Dieser ist gemäß dem hier vorgestellten Ausführungsbeispiel als nichtdispersiver Infrarotdetektor ausgeformt. Der Messgassensor 5 weist eine Einlassöffnung 6 sowie eine Auslassöffnung 7 auf. Innerhalb des Messgassensors 5 ist ein in der schematischen Darstellung gemäß Fig. 1 nicht ausdrücklich gezeigter Messraum angeordnet.

Ferner ist im Innern des Gehäuses 3 eine über den für das im Wasser 2 enthaltene, seinerseits beispielsweise Kohlendioxid als Messgas enthaltende, Umgebungsgas durchlässigen Membranequilibrator 4 mit der Umgebung verbundene Einlassleitung 8 für das Umgebungsgas angeordnet.

Die Einlassleitung 8 mündet in die Einlassöffnung 6 des Messgassensors 5. In der Einlassleitung 8 ist eine Pumpe 9 sowie stromabwärts der Pumpe 9 eine Heizvorrichtung 10 zum Beheizen des durch den Membranequilibrator 4 in die Einlassleitung 8 gelangten Umgebungsgases angeordnet. Stromabwärts der Heizvorrichtung 10 sind in der Einlassleitung 8 nacheinander ein Sensor 11 für Temperatur und Feuchtigkeit sowie ein Drucksensor 12 angeordnet. Die Auslassöffnung 7 des Messgassensors 5 mündet in eine Auslassleitung 13, welche über den Membranequilibrator 4 mit dem umgebenden Wasser 2 kommuniziert. In der Auslassleitung 13 ist ebenfalls ein Drucksensor 14 sowie ein Temperatur- und Feuchtigkeitssensor 15 angeordnet.

Stromaufwärts von der Pumpe 9 ist in der Einlassleitung 8 ein Ventil 16 angeordnet. Das Ventil 16 ist so konfiguriert, dass es im ersten Schaltzustand eine Verbindung der Saugseite der Pumpe 9 mit einem dem Membranequilibrator 4 zugewandten Abschnitt der Einlassleitung 8 herstellt. Dieser Schaltzustand ist in der Figur 1 skizziert. Im zweiten möglichen Schaltzustand des Ventils, welchen das Ventil 16 einnehmen kann, ist die Saugseite der Pumpe 9 der Einlassleitung 8 mit einer Nullgasquelle 17 verbunden, so dass der Abschnitt der Einlassleitung 8 zwischen dem Membranequilibrator 4 und dem Ventil 16 nicht mit der Ansaugseite der Pumpe 9 kommuniziert. In diesem Abschnitt der Einlassleitung 8 sind ebenfalls ein Sensor 18 für Temperatur und Feuchtigkeit sowie ein Drucksensor 19 vorgesehen.

In der Auslassleitung 13 ist stromaufwärts des Sensors 15 für Temperatur und Feuchtigkeit sowie des Drucksensors 14 ein Ventil 20 angeordnet. Das Ventil 20 in der Auslassleitung 13 ist analog zu dem Ventil 16 der Einlassleitung 8 aufgebaut und kann ebenfalls zwei mögliche Schaltzustände einnehmen. Dabei ist der erste mögliche Schaltzustand des Ventils 20 in der Auslassleitung 13 der in der Figur dargestellte, bei welchem die Auslassöffnung 7 des Messgassensors 5 über das Ventil 20 und den Membranequilibrator 4 mit dem umgebenden Wasser 2 kommuniziert. In dem zweiten möglichen Schaltzustand des Ventils 20 in der Auslassleitung 13 ist demgegenüber die Auslassöffnung 7 des Messgassensors 5 mit einem Eingang 21 der Nullgasquelle 17 verbunden, wobei in diesem Schaltzustand die Auslassöffnung 7 des Messgassensors 5 nicht über den Membranequilibrator 4 mit dem umgebenden Wasser 2 verbunden ist. In diesem zweiten möglichen Schaltzustand des Ventils 20 in der Auslassleitung 13 ist bei entsprechender Stellung des Ventils 16 in der Einlassleitung 8 eine die Einlassöffnung 6 mit der Auslassöffnung 7 des Messgassensors 5 verbindende Bypass-Leitung geschaltet, wobei die Bypass-Leitung in diesem Schaltzustand der Ventile 20, 16 über das Ventil 20 in der Auslassleitung 13, die Nullgasquelle 17, das Ventil 16 in der Einlassleitung 8, die Pumpe 9 und die Heizvorrichtung 10 die Auslassöffnung 7 mit der Einlassöffnung 6 des Messgassensors 5 verbindet. In diesem Schaltzustand zirkuliert Gas durch den Messgassensor 5 innerhalb des Gehäuses 3 des Partialdrucksensors 1, ohne dass ein Gasaustausch über den Membranequilibrator 4 mit dem umgebenden Wasser 2 erfolgt.

Schließlich umfasst der Partialdrucksensor 1 gemäß Figur 1 eine nur schematisch gezeigte Steuer- und Auswerteeinheit 22. Die Steuer- und Auswerteeinheit 22 ist über eine Steuerleitung 23 mit den Ventilen 16, 20 in der Auslassleitung 13 bzw. in der Einlassleitung 8 verbunden, um die Ventile 16, 20 zwischen den beschriebenen möglichen Schaltzuständen zu schalten. Außerdem ist die Steuer- und Auswerteeinheit 22 über eine Signalleitung 24 mit Auslesemitteln 25 zum Auslesen des Messgassensors 5 verbunden. Schließlich ist die Steuer- und Auswerteeinheit 22 über weitere, in der Figur nicht dargestellte Signalleitungen mit den Sensoren 18, 19, 11, 12, 14, 15 für Temperatur, Feuchtigkeit beziehungsweise Druck verbunden, um anhand des vom optischen Messgassensor 5 erfassten Absorptionssignals den Partialdruck zu bestimmen.

Die Steuer- und Auswerteeinheit 22 weist Mittel zum Speichern der über die Auslesemittel 25 von dem Messgassensor 5 ausgelesenen Messwerte auf. Außerdem sind in der Steuer- und Auswerteeinheit Mittel zum Analysieren und Aufbereiten der Messdaten vorgesehen.

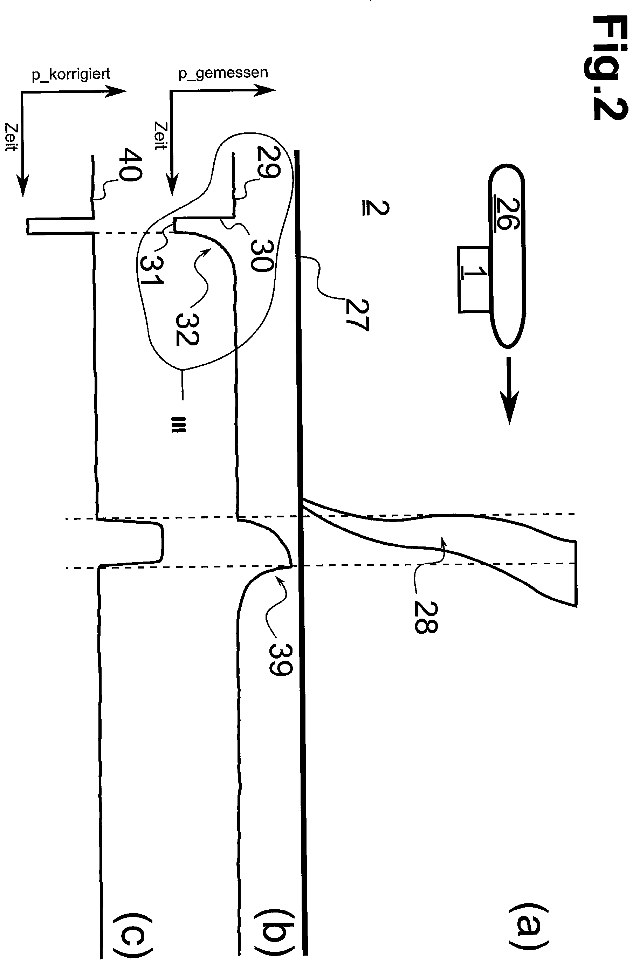

Die Figur 2 veranschaulicht in schematischer Darstellung eine bevorzugte Ausführungsform des erfindungsgemäßen Verfahrens zum Betreiben des Partialdrucksensors 1 gemäß Figur 1. Im Teil a) der Figur 2 ist schematisch ein Geräteträger 26 dargestellt, welcher z.B. ein AUV (Autonomous Unterwater Vehicle), ROV (Remotely Operated Vehicle), ein Schiff oder ähnliches sein kann. An dem Geräteträger 26 ist der Partialdrucksensor 1 gemäß Figur 1 befestigt. Wie in der Figur 2A durch einen Pfeil angedeutet, bewegt sich der Geräteträger 26 mit dem daran befestigten erfindungsgemäßen Partialdrucksensor 1 über dem Meeresboden 27 durch das umgebende Wasser in Richtung des Pfeils. In der Fortbewegungsrichtung des Geräteträgers 26 ist schematisch ein Bereich 28 mit erhöhter Messgaskonzentration angedeutet. Beispielsweise handelt es sich dabei um Kohlendioxid, welches aus einer Quelle im Meeresboden 27 in das Wasser 2 austritt. Räumliche Lage und Ausdehnung sowie Partialdruck des Bereichs 28 mit erhöhter Messgaskonzentration sollen nun mit dem am Geräteträger 26 angeordneten Partialdrucksensor 1 möglichst genau vermessen werden, indem der Geräteträger 26 durch den Bereich 28 mit einer bestimmten Geschwindigkeit bewegt wird.

Um der bei dieser Messung von dem bewegten Geräteträger 26 aus auftretende Problematik im Zusammenhang mit einer endlichen Ansprechzeit des Partialdrucksensors 1 auf Änderungen im Messsignal, also Änderungen in dem Partialdruck des Messgases, entgegenzuwirken, wird erfindungsgemäß, während sich der Geräteträger 26 mit dem Partialdrucksensor 1 in einem Bereich ausreichender Entfernung von dem Bereich 28 mit erhöhter Messgaskonzentration bewegt, in welchem ein gleichbleibender Partialdruck des Messgases herrscht, zunächst eine Bestimmung der Ansprechcharakteristik des Partialdrucksensors 1 wie folgt durchgeführt.

Zunächst wird, wie in Figur 1 veranschaulicht, über die Steuer- und Auswerteeinheit 22 durch Schalten des Ventils 20 in der Auslassleitung 13 sowie des Ventils 16 und der Einlassleitung 8 eine Bypass-Leitung zwischen der Auslassöffnung 7 und der Einlassöffnung 6 des Messgassensors 5 geschaffen, welche durch die Nullgasquelle 17 geführt ist. Das durch die Nullgasquelle 17 geleitete Umgebungsgas wird in der Nullgasquelle anschließend in Nullgas umgewandelt. In dem hier betrachteten Beispiel, bei dem das Messgas Kohlendioxid ist, erfolgt die Umwandlung durch Durchgang des Umgebungsgases durch eine Säule aus Natronkalk. Bei einer Ausführung der Erfindung, bei welcher Methan das Messgas ist, kann die Umwandlung durch Oxidation mit dem im Umgebungsgas enthaltenen Sauerstoff erfolgen, um $CO_2$ und $H_2O$ an einem thermokatalytischen Element, das in der Form eines thermokatalytischen Sensors als Pellistor vorliegen kann, zu erzeugen.

Der Messgassensor 5 ist gemäß dem Ausführungsbeispiel nur auf $CO_2$ empfindlich. Nach Durchgang durch die Nullgasquelle 17 tritt somit Nullgas, also Umgebungsgas, welches kein CO2 enthält, in das Ventil 16 in der Einlassleitung 8 und wird über die Pumpe 9 und die Heizvorrichtung 10 durch die Einlassleitung 6 in den Messraum des Messgassensors 5 eingeleitet. In Figur 2B ist eine über die Auslesemittel 25 von der Steuer- und Auswerteeinheit 22 aus dem Messgassensor 5 ausgelesene zeitliche Reihe 29 aufgezeichneter Messdaten dargestellt. Die zeitliche Reihe 29 ist in Fig. 2B derart unterhalb der räumlichen Skizze gemäß Figur 2A angeordnet, dass ersichtlich ist, welchem Messort, also welcher Position des am Geräteträger 26 befestigten Partialdrucksensors 1, ein Messpunkt der zeitlichen Reihe 29 entspricht.

In der zeitlichen Reihe 29 gemäß Figur 2B ist nun ersichtlich, dass die beschriebene Durchströmung des Messraums mit dem in der Nullgasquelle 17 erzeugten Nullgas zu einem sprunghaften Abfall 30 des Messsignals in der zeitlichen Reihe 29 führt. Weiter ist ersichtlich, dass das Messsignal während der Durchströmung des Messraums des Messgassensors 5 mit dem Nullgas ein Nullplateau 31 einnimmt. Nachdem das Nullplateau 31 erreicht wurde, entspricht die zeitliche Reihe 29 des Messsignals dem tatsächlichen Partialdruck in dem Messraum des Messgassensors 5, da ein stationärer Zustand erreicht ist, an dem etwaige Effekte bedingt durch ein verzögertes Ansprechverhalten keine Rolle mehr spielen. Das Nullplateau 31 kann außerdem, wie für sich genommen bekannt, zur Kalibrierung des Messgassensors 5 verwendet werden, um eine etwaige Nullpunktsverschiebung des Messgassensors zu korrigieren.

Weiter ist in der zeitlichen Reihe 29 der aufgezeichneten Messdaten gemäß Figur 2B der sich an das Nullplateau 31 anschließende flankenartige Anstieg 32 des Messsignals zu erkennen, welcher aufgezeichnet wird, wenn das Ventil 16 in der Einlassleitung 8 sowie das Ventil 20 in der Auslassleitung 13 so geschaltet werden, dass die Nullgasquelle 17 nicht mehr mit dem Messraum des Messgassensors 5 verbunden ist und stattdessen eine Durchströmung des Messgassensors 5 mit über den Membranequilibrator 4 in die Einlassleitung 8 gezogenem Umgebungsgas erfolgt.

Da sich, wie der Vergleich mit Figur 2A verdeutlicht, in dem zu dem flankenartigen Anstieg 32 der zeitlichen Reihe 29 entsprechenden räumlichen Segment der Partialdruck des Messgases, beispielhaft Kohlendioxid, nicht ändert, müsste sich anstelle des flankenartigen Anstiegs 32 bei einer idealen Messung ein sprunghafter Anstieg an das Nullplateau 31 anschließen. Dass stattdessen der flankenartige Anstieg 32 aufgezeichnet wird, ist auf die Ansprechcharakteristik des Partialdrucksensors 1 zurückzuführen, welches durch sämtliche Komponenten des Partialdrucksensors 1 beeinflusst wird. Dazu gehören insbesondere der Membranequilibrator 4, das Ventil 16 in der Einlassleitung 8, die Pumpe 9, die Heizvorrichtung 10, der Messgassensor 5, das Ventil 20 in der Auslassleitung 13. Da bei dem erfindungsgemäßen Durchströmen des Messgassensors 5 *in situ* mit Nullgas bei im Wesentlichen konstantem Partialdruck des Messgases, beispielsweise *Kohlendioxid,* die reale Messgröße sprunghaft ansteigt, lässt sich durch Auswertung des flankenartigen Anstiegs 32 die Ansprechcharakteristik des Messgassensors 5 bestimmen.

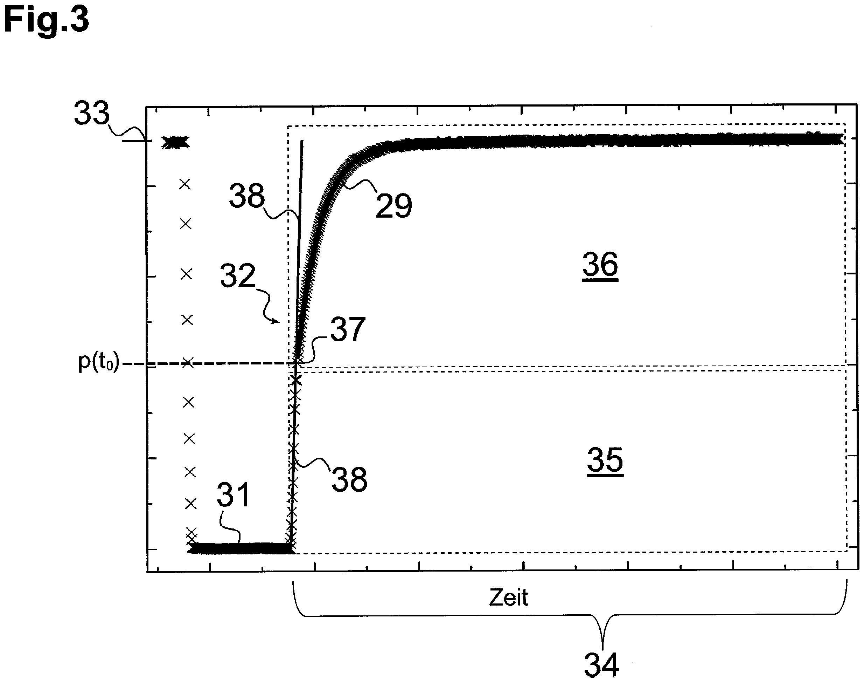

Die Bestimmung der Ansprechcharakteristik durch Auswertung des flankenartigen Anstiegs 32 ist in Figur 3 näher erläutert. Die Figur 3 zeigt eine Vergrößerung des Bereichs III der zeitlichen Reihe 29 gemäß Figur 2B zwecks Veranschaulichung des erfindungsgemäßen Verfahrens zur Bestimmung der Ansprechcharakteristik des Messgassensors 5. Wie in Figur 3 zu erkennen, zeigt die Messreihe zunächst den Ausgangspartialdruck 33 des Messgases im Wasser 2, welcher an der in Figur 2A gezeigten Position des Geräteträgers 26 und damit des Partialdrucksensors 1 herrscht. Im Bereich des Nullplateaus 31 verschwindet der gemessene und tatsächliche Partialdruck in etwa.

Zur Auswertung der Ansprechcharakteristik anhand des flankenartigen Anstiegs 32 der zeitlichen Reihe 29 wird erfindungsgemäß nach dem hier beschriebenen Ausführungsbeispiel wie folgt vorgegangen. Der Auswertung des flankenartigen Anstiegs 32 wird eine Wertereihe innerhalb eines Kalibrationsintervalls 34 zugrundegelegt, währenddessen der Partialdruck des Messgases in der Umgebung konstant ist, wie in Figur 2A zu erkennen. Zunächst wird der Bereich des flankenartigen Anstiegs 32 analytisch in einen ersten Abschnitt 35, welcher während einer Durchmischungsphase des Messgases mit dem Nullgas in dem Messraum des Messgassensors 5 aufgenommen wurde, und einen zweiten Abschnitt 36 aufgeteilt. Dabei entsprechen dem ersten Abschnitt 35 der Messreihe 29 Werte, die sich unmittelbar an das Nullplateau 31 anschließen, bis zu einem Übergangspunkt 37. Entsprechend erstreckt sich der zweite Abschnitt 36 der Messreihe 29 über Messpunkte von dem Übergangspunkt 37 bis zum letzten Messpunkt innerhalb des Kalibrationszeitintervalls 34.

Zur Bestimmung des Übergangspunkts 37 wird erfindungsgemäß eine Regressionsgerade 38 ausgehend von den ersten Messwerten der Messreihe 29, welche sich an das Nullplateau 31 anschließen, bestimmt. Der Übergangspunkt 37 ergibt sich dann durch Bestimmung des Punktes, an welchem die Messwerte die Regressionsgerade 38 verlassen. Analytisch ist dies der Punkt, an welchem das Residuum zwischen den tatsächlichen Messwerten und entsprechenden Werten auf der Regressionsgerade 38 einen bestimmen Wert übersteigt.

Im nächsten Schritt wird unter Berücksichtigung nur des zweiten Abschnitts 36 der Messreihe 29 eine Regressionsanalyse durchgeführt, indem die Messwerte innerhalb des zweiten Abschnitts der Messreihe 36 an folgendes Modell angepasst werden:

$$p_{(t)} = p_{water} + (p_{(t_0)} - p_{water})e^{-\frac{t}{\tau}},$$

wobei $p_{(t)}$ Messwerten der zeitlichen Reihe 29 im zweiten Abschnitt 36 entspricht, $p_{water}$ dem Ausgangspartialdruck 33 des Messgases entspricht und $p_{(t_0)}$ das Ausgangssignal des gemessenen Partialdrucks zum Zeitpunkt $t_0$, also am Übergangspunkt 37, entspricht und wobei $\tau$ eine im Rahmen der Regressionsanalyse zu bestimmende Zeitkonstante bezeichnet.

Mit dem beschriebenen Verfahren ist erfindungsgemäß die Ansprechcharakteristik des Partialdrucksensors 1 *in situ* bestimmt. Die Regressionsparameter, insbesondere der Parameter $\tau$, berücksichtigen implizit mit großem Vorteil sämtliche die Ansprechcharakteristik des Partialdrucksensors 1 am Ort der Messung beeinflussende Faktoren, wie z. B. Temperatur, Druck, Eigenschaften des Membranequilibrators 4 etc.

Mit Bezug auf Figur 2A wird nachfolgend der weitere Ablauf der Messung veranschaulicht. Der Geräteträger 26 bewegt sich, nachdem mit dem beschriebenen Verfahren die Ansprechcharakteristik bestimmt worden ist, durch den Bereich 28 mit erhöhter Messgaskonzentration. Dabei wird in der zeitlichen Reihe 29 der aufgezeichneten Messdaten eine Rohmessantwort 39 aufgezeichnet. Diese ist zwangsläufig durch die Ansprechcharakteristik des Partialdrucksensors 1 verfälscht. Aufgrund der mit Bezug auf die Figur 3 im Einzelnen beschriebenen Bestimmung der Ansprechcharakteristik ermöglicht das erfindungsgemäße Verfahren nun die rechnerische Kompensation der Messdaten der zeitlichen Reihe 29. Dies erfolgt gemäß dem hier beschriebenen Ausführungsbeispiel anhand der Formel

$$p_{water} = \frac{p_{(t)} - p_{(t_0)}e^{-\frac{t}{\tau}}}{1 - e^{-\frac{t}{\tau}}}$$

Bei einer typischerweise diskret aufgezeichneten Messreihe 29 erfolgt die Auswertung dieser Formel in der Praxis durch folgende iterative Formel:

$$p_{corrected,t_{i+1}} = \frac{p_{(t_{i+1})} - p_{(t_i)}e^{-\frac{\Delta t}{\tau}}}{1 - e^{-\frac{\Delta t}{\tau}}} \quad \text{mit} \quad \Delta t = t_{i+1} - t_i$$

Dabei bezeichnen die Zeitpunkte $t_{(i+1)}$ bzw. $t_i$ zeitlich aufeinanderfolgende Werte der zeitlichen Reihe 29, wobei darauf geachtet werden muss, dass $\Delta t$, also die Abtastrate, so klein gewählt ist, dass der Partialdruck des Messgases im Wasser 2 innerhalb dieses Zeitintervalls als konstant angenommen werden kann. Dies richtet sich also nach dem Partialdruckgradienten im Wasser 2.

Wendet man nun die genannte Korrektur auf die zeitliche Reihe 29 der über die Auslesemittel 25 des Messgassensors 5 aufgezeichneten Messdaten an, erhält man die in Figur 2C gezeigte korrigierte Messreihe 40, welche eine korrekte zeitliche und damit räumliche Zuordnung der Messwerte zu den in Figur 2A veranschaulichten Messbedingungen hinsichtlich des Partialdrucks des Messgases wiedergibt. Insbesondere ist zu erkennen, dass im Anfangsbereich eine rechteckige Sprungfunktion angezeigt ist, welche gemäß dem oben beschriebenen Zeroing Vorgang entspricht. Außerdem ist zu erkennen, wie eine räumlich zutreffende Zuordnung der Messdaten zu dem Bereich 28 mit erhöhter Messgaskonzentration, insbesondere der Rohmessantwort 39, erfolgt.

Der Fachmann wird erkennen, dass die Datenanalyse, insbesondere die Regressionsanalyse, geeignete, allgemein übliche Datenfilterung voraussetzt.

Anhand der Figuren ist somit eine erfindungsgemäße Vorrichtung sowie ein erfindungsgemäßes Verfahren zum Betreiben derselben beschrieben, welche anhand eines *in situ* Zeroing, also einer *in situ* Bestimmung des Ansprechverhaltens des Partialdrucksensors 1, eine verbesserte Messung von Messgas in Umgebungsgas ermöglicht. Die Verbesserung bezieht sich insbesondere auf eine verbesserte räumliche bzw. zeitliche Zuordnung der Messwerte zu den realen Partialdruckbedingungen.

Das beschriebene Durchströmen des Messraums des Messgassensors 5 durch entsprechendes Schalten der Ventile 16, 20 und die sich daran anschließende Bestimmung des Ansprechverhaltens kann erfindungsgemäß mehrmals *in situ* durchgeführt werden, insbesondere nachdem sich Umgebungsparameter geändert haben. Eine Umrechnung der zeitlichen Reihe 29 anhand der Ansprechcharakteristik zu den tatsächlichen Werten 40 gemäß Figur 2C erfolgt dann jeweils auf Basis der zuletzt bestimmten Ansprechcharakteristik.

Zwischen derartigen Bestimmungen der Ansprechcharakteristik kann im Rahmen der Erfindung auch eine rechnerische Korrektur der Ansprechcharakteristik vorgenommen werden anhand von in dem umgebenden Wasser 2, also außerhalb des Partialdrucksensors 1, gemessenen Werten für Temperatur, Feuchtigkeit sowie Druck, wenn ein entsprechendes Modell zur Abhängigkeit der Ansprechcharakteristik von diesen Parametern zugrunde gelegt wird.

Das mit Bezug auf die Figuren 2 und 3 beschriebene Verfahren beruht implizit darauf, dass das Umgebungsgas am Ort der Durchführung des erfindungsgemäßen Zeroing-Verfahrens Messgas mit einem nicht verschwindenden Ausgangspartialdruck 33 enthält. Da es sich bei dem Partialdrucksensor 1 um einen mit einem Messgassensor 5 für $CO_2$ versehenen Partialdrucksensor handelt, welcher eben zur Detektion von $CO_2$ in Umgebungsgas ausgelegt ist, ist diese Bedingung bei dem in den Figuren beschriebenen Einsatz des Partialdrucksensors 1 in Wasser 2 in aller Regel der Fall. Denn in Wasser gelöste Umgebungsluft weist normalerweise einen oberhalb des Detektionslimits des Messgassensors 5 liegenden Partialdruck für $CO_2$ auf.

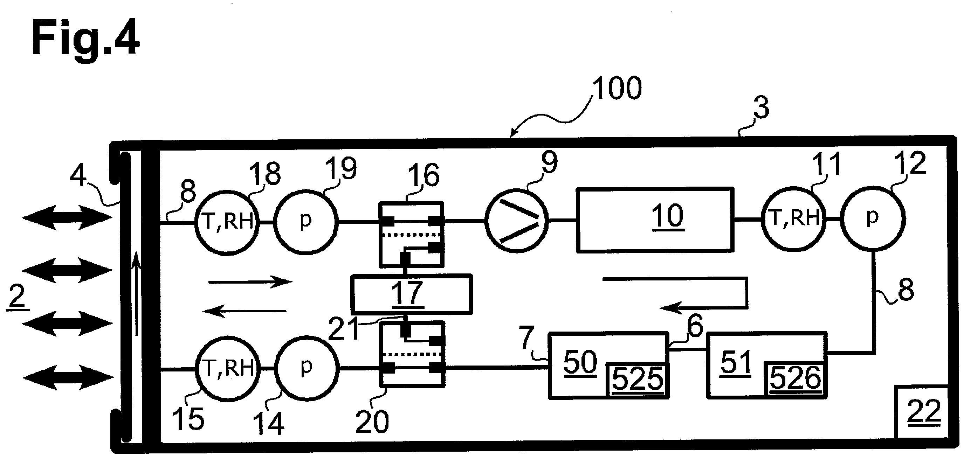

Soll das erfindungsgemäße Zeroing-Verfahren hingegen an einem Ort durchgeführt werden, an welchem das Messgas innerhalb des Umgebungsgases einen zu geringen Ausgangspartialdruck 33 aufweist, welcher kleiner als das Detektionslimit des Messgassensors 5 ist, kann das mit Bezug auf Figur 3 beschriebene Auswerteverfahren für die Ansprechcharakteristik nicht oder nicht mit befriedigenden Ergebnissen durchgeführt werden. In derartigen Fällen kann im Rahmen der Erfindung ein Verfahren gemäß einer Weiterbildung der Erfindung mit dem in Figur 4 schematisch gezeigten Partialdrucksensor 100 durchgeführt werden. Der Partialdrucksensor 100 gemäß Figur 4 ist zur Detektion des Partialdrucks von Methangas als Messgas im Umgebungsgas ausgelegt. Methangas wird hingegen in vielen Fällen im Umgebungsgas normalerweise nicht oberhalb eines Detektionslimits vorhanden sein.

Der Partialdrucksensor 100 ist im Wesentlichen analog zu dem Partialdrucksensor 1 gemäß Figur 1 aufgebaut. Jedoch ist anstelle des Messgassensors 5 für CO2, der beim Partialdrucksensor gemäß Figur 1 eingesetzt ist, ein Messgassensor 50 angeordnet, welcher für Methangas als Messgas im Umgebungsgas empfindlich ist. Dem für Methangas empfindlichen Messgassensor 50 ist stromaufwärts in der Einlassleitung 8 ein Korrekturgassensor 51 in Serie vorgeschaltet. Der Korrekturgassensor 51 ist im Prinzip ein Sensor analog den Messgassensoren 5, 50. Jedoch ist der Korrekturgassensor 51 für CO2 Gas als Messgas im Umgebungsgas empfindlich.

Wird nun das Zeroing-Verfahren durch geeignetes Schalten der Ventile 16, 20 zur Durchströmung der Nullgasquelle 17 durchgeführt, führt dies, wie oben ausführlich beschrieben, dazu, dass das durch die Einlassleitung 8 und daher den Korrekturgassensor 51 und den Messgassensor 50 für CH4 als Messgas mit einem Gas durchströmt wird, dem aufgrund des Durchlaufens der Nullgasquelle 17 der CO2-Anteil entzogen wurde und welches darüber hinaus von vornherein kein Methangas (CH4) enthält. Für das Messsignal des Messgassensors 50, welcher ausschließlich auf Methangas als Messgas reagiert, führt dies zu keiner Änderung. Denn vor, während und nach dem Zeroing enthält das dem Messgassensor 50 zugeführte Gas kein Methangas. Das Messsignal, welches vom Korrekturgassensor 51 aufgezeichnet wird, zeigt demgegenüber das mit Bezug auf Figur 3 ausführlich erläuterte Verhalten, welches eine Bestimmung des Ansprechverhaltens zulässt. Aufgrund einer, beispielsweise unter Laborbedingungen durchgeführten, Kalibrierung lässt sich dann von der auf die beschriebene Weise ermittelten Korrekturansprechcharakteristik des Nullgassensors 51 auf die gesuchte Ansprechcharakteristik des Partialdrucksensors 100 schließen.

Wenngleich in Figur 4 schematisch zwei in Serie geschaltete Gassensoren 50, 51 gezeigt sind, kann gleichermaßen ein Gassensor eingesetzt werden, bei welchem neben dem Detektor für das Messgas, in diesem Beispiel Methan, ein weiterer Detektor für das Nullgas, hier das CO2-freie Gas, verwendet werden, welcher innerhalb ein und derselben Messkammer den Partialdruck des Nullgases misst.

BEZUGSZEICHENLISTE

| 1 | Partialdrucksensor |
|---|---|
| 2 | Wasser |
| 3 | Gehäuse |
| 4 | Membranequilibrator |
| 5 | Messgassensor für CO2 |
| 6 | Einlassöffnung |
| 7 | Auslassöffnung |
| 8 | Einlassleitung |
| 9 | Pumpe |
| 10 | Heizvorrichtung |
| 11 | Sensor für Temperatur und Feuchtigkeit |
| 12 | Drucksensor |
| 13 | Auslassleitung |
| 14 | Drucksensor |
| 15 | Sensor für Temperatur und Feuchtigkeit |
| 16 | Ventil |
| 17 | Nullgasquelle |
| 18 | Sensor für Temperatur und Feuchtigkeit |
| 19 | Drucksensor |
| 20 | Ventil |
| 21 | Eingang |
| 22 | Steuer- und Auswerteeinheit |
| 23 | Steuerleitung |
| 24 | Signalleitung |
| 25 | Auslesemittel |
| 26 | Geräteträger |
| 27 | Meeresboden |
| 28 | Bereich mit erhöhter Messgaskonzentration |

| 29 | zeitliche Reihe über die Auslesemittel aufgezeichneter Messdaten |
| 30 | sprunghafter Abfall |
| 31 | Nullplateau |
| 32 | flankenartiger Anstieg |
| 33 | Ausgangspartialdruck |
| 34 | Kalibrationszeitintervall |
| 35 | erster Abschnitt der Messreihe |
| 36 | zweiter Abschnitt der Messreihe |
| 37 | Übergangspunkt |
| 38 | lineare Regressionsgerade |
| 39 | Rohmessantwort |
| 40 | korrigierte Messseite |
| 50 | Messgassensor für CH4 |
| 51 | Korrekturgassensor für CO2 |
| 100 | Partialdrucksensor für CH4 |
| 525 | Auslesemittel |
| 526 | Auslesemittel |

**Patentansprüche**

1. Vorrichtung (1) zur Detektion eines Partialdrucks eines in einem Umgebungsgas enthaltenen Messgases, umfassend einen Messraum mit mindestens einer Einlassöffnung (6) zum Einlassen von Umgebungsgas in den Messraum und mindestens einer Auslassöffnung (7) zum Auslassen von Umgebungsgas aus dem Messraum, einen auf das Messgas empfindlichen Messgassensor (5) zum Messen einer für den Partialdruck des Messgases im Messraum kennzeichnenden Messgröße sowie Auslesemittel (25) zum Auslesen des Messgassensors (5), wobei Mittel zur Bestimmung einer Ansprechcharakteristik (32) der Vorrichtung (1) vorgesehen sind, **dadurch gekennzeichnet, dass** die Mittel zur Bestimmung einer Ansprechcharakteristik Mittel (16, 17, 20) zum Durchströmen des Messraumes mit einem Nullgas, auf welches der Messgassensor (5) und/oder der Korrekturgassensor (51) nicht empfindlich ist, umfassen und dass eine Steuer- und Auswerteeinheit (22) zur Steuerung der Mittel (16, 17, 20) zum Durchströmen des Messraumes vorgesehen ist, um ein Durchströmen des Messraumes mit einem Nullgas gezielt ein- und auszuleiten, wobei die Steuer- und Auswerteeinheit (22) zur rechnerischen Bestimmung einer Ansprechcharakteristik (32) aus einer zeitlichen Reihe (29) über die Auslesemittel (25) des Messgassensors (5) und/oder über die Auslesemittel (526) das Korrekturgassensors (51) aufgezeichneter Messdaten ausgebildet ist und/oder zur rechnerischen Kompensation einer Ansprechcharakteristik (32) der über die Auslesemittel (5) des Messgassensors (5) aufgezeichneten Messdaten ausgestaltet ist.

2. Vorrichtung (1) nach Anspruch 1, **dadurch gekennzeichnet, dass** die Mittel zur Bestimmung einer Ansprechcharakteristik einen auf ein Korrekturgas empfindlichen Korrekturgassensor (51) zum Messen einer für einen Partialdruck des Korrekturgases im Messraum kennzeichnenden Messgröße sowie Auslesemittel (256) zum Auslesen des Korrekturgassensors (51) umfassen, wobei das Korrekturgas in dem Umgebungsgas enthalten und/oder von dem Messgas verschieden ist.

3. Vorrichtung (1) nach Anspruch 1 oder 2, **dadurch gekennzeichnet, dass** sie eine Nullgasquelle (17) zur Bereitstellung des Nullgases aufweist, wobei die Nullgasquelle (17) zur Erzeugung des Nullgases aus dem Umgebungsgas ausgebildet ist und/oder Mittel zum Entfernen des Messgases aus dem Umgebungsgas umfasst und/oder dass die Nullgasquelle (17) Mittel zum chemischen Umwandeln des Messgases derart umfasst, dass der Messgassensor (5) und/oder der Korrekturgassensor (51) auf bei der Umwandlung entstehende gasförmige Umwandlungsprodukte nicht empfindlich ist.

4. Vorrichtung (1) nach einem der vorhergehenden Ansprüche, **dadurch gekennzeichnet, dass** stromaufwärts von der Einlassöffnung (6) und/oder stromabwärts von der Auslassöffnung (7) eine für das Umgebungsgas durchlässige Membran (4) derart angeordnet ist, dass das Umgebungsgas durch die Membran (4) in den Messraum gelangt und/oder das Umgebungsgas die Vorrichtung durch die Membran (4) verlässt.

5. Vorrichtung (1) nach einem der vorhergehenden Ansprüche, **dadurch gekennzeichnet, dass** eine die Einlassöffnung(6) mit der Auslassöffnung (7) verbindende Bypass-Leitung vorgesehen ist.

**6.** Vorrichtung (1) nach einem der vorhergehenden Ansprüche, **dadurch gekennzeichnet, dass** der Messgassensor (5) und/oder der Korrekturgassensor (51) einen optischen Gassensor, einen nicht-dispersiven Infrarotsensor und/oder ein auf einer durchstimmbaren Laserdiode basierendes Spektrometer und/oder einen auf einer durchstimmbaren Laserdiode basierenden Sensor, umfasst und/oder dass eine Pumpe (9) vorgesehen ist, um Umgebungsgas durch den Messraum zu zirkulieren.

**7.** Verfahren zum Betreiben einer einen Messraum und einen auf ein in einem Umgebungsgas enthaltenes Messgas empfindlichen Messgassensor (5) zum Messen einer für den Partialdruck des Messgases im Messraum kennzeichnenden Größe umfassenden Vorrichtung (1), **dadurch gekennzeichnet, dass** es ein Verfahren zur Bestimmung einer Ansprechcharakteristik (32) der Vorrichtung (1) umfasst, bei welchem

a. der Messraum mit einem Nullgas, auf welches der Messgassensor (5) und/oder ein auf ein Korrekturgas empfindlicher Korrekturgassensor (51) nicht empfindlich ist, durchströmt wird, wobei gleichzeitig ein Ausgangssignal des Messgassensors (5) und/oder ein Ausgangssignal des Korrekturgassensors (51) beobachtet wird, bis dieses einen im wesentlichen gleichbleibenden Minimalwert erreicht;
b. der Messraum mit Umgebungsgas durchströmt wird, nachdem das Ausgangssignal den Minimalwert erreicht hat, wobei mit Beginn der Durchströmung mit Umgebungsgas das Ausgangssignal des Messgassensors (5) und/oder das Ausgangssignal des Korrekturgassensors (51) während eines Kalibrationszeitintervalls (34) zeitaufgelöst in einer Messreihe aufgezeichnet wird;
c. anhand einer Auswertung der Messreihe die Ansprechcharakteristik bestimmt wird,

wobei zumindest die Schritte (a) und (b) *in situ* durchgeführt werden.

**8.** Verfahren nach Anspruch 7, **dadurch gekennzeichnet**, **d a s** s zur Auswertung der Messreihe zunächst eine Korrekturansprechcharakteristik auf Basis des Ausgangssignals des Korrekturgassensors (51) bestimmt wird und anschließend aus der Korrekturansprechcharakteristik die Ansprechcharakteristik der Vorrichtung bestimmt wird.

**9.** Verfahren nach Anspruch 7 oder 8, **dadurch gekennzeichnet, dass** das Ausgangssignal des Messgassensors (5) anhand der Ansprechcharakteristik korrigiert wird, um eine zeitliche Zuordnung des Ausgangssignals zu dem Partialdruck des Messgases zum Zeitpunkt der Aufnahme des Ausgangssignals zu erhalten,

**10.** Verfahren nach einem der Ansprüche 7 bis **9,dadurch gekennzeichnet, dass** das Kalibrationszeitintervall (34) so gewählt wird, dass der Partialdruck des Messgases und/oder der Partialdruck des Korrekturgases während des Kalibrationszeitintervalls (34) im wesentlichen konstant ist.

**11.** Verfahren nach einem der Ansprüche 7 bis 10, **dadurch gekennzeichnet, dass** für die Bestimmung der Ansprechcharakteristik ein erster Abschnitt (35) der Messreihe, welcher während einer Durchmischungsphase des Umgebungsgases mit dem Nullgas in dem Messraum aufgezeichnet wurde, nicht berücksichtigt wird, wobei sich der erste Abschnitt (35) der Messreihe bis zu einem Übergangspunkt (37) von einem im wesentlichen linearen zu einem im wesentlichen exponentiellen Kurvenverlauf erstreckt.

**12.** Verfahren nach einem der Ansprüche 7 bis 11, **dadurch gekennzeichnet, dass** die Ansprechcharakteristik anhand einer Regressionsanalyse der Messreihe (29) bestimmt wird, wobei vorzugsweise folgendes Modell angesetzt wird:

$$p_{(t)} = p_{water} + (p_{(t_0)} - p_{water})e^{-\frac{t}{\tau}}$$

wobei p(t) das Ausgangssignal zum Zeitpunkt t, $p_{water}$ den Partialdruck des Messgases innerhalb des Umgebungsgases, $p(t_0)$ das Ausgangssignal zum Zeitpunkt $t_0$ und $\tau$ eine Zeitkonstante bezeichnen.

**13.** Verfahren nach einem der Ansprüche 7 bis 12, **dadurch gekennzeichnet, dass** das Ausgangssignal des Messgassensors (5) anhand der Ansprechcharakteristik korrigiert wird, indem das Ausgangssignal p(t) zum Zeitpunkt t dem Partialdruck $p_{water}$ des Messgases innerhalb des Umgebungsgases zum Zeitpunkt der Aufnahme des Ausgangssignals gemäß folgender Formel zugeordnet wird:

$$p_{water} = \frac{p_{(t)} - p_{(t_0)}e^{-\frac{t}{\tau}}}{1 - e^{-\frac{t}{\tau}}},$$

wobei $p(t_0)$ das Ausgangssignal zum Zeitpunkt $t_0$ bezeichnet.

**14.** Verfahren nach einem der Ansprüche 7 bis 13, **dadurch gekennzeichnet, dass** das Verfahren zur Bestimmung einer Ansprechcharakteristik mehrmals, nach jeder signifikanten Änderung eines oder mehrerer Umgebungsparameter, durchgeführt wird.

**Claims**

**1.** Device (1) for detecting a partial pressure of a measurement gas that is contained in a surrounding gas, said device comprising a measurement chamber which has at least one inlet opening (6) for admitting surrounding gas into the measurement chamber and at least one outlet opening (7) for discharging surrounding gas from the measurement chamber, and comprising a measurement gas sensor (5) which is sensitive to the measurement gas and is intended for measuring a measurement variable characterising the partial pressure of the measurement gas in the measurement chamber, and reading means (25) for reading the measurement gas sensor (5), means for determining a response characteristic (32) of the device (1) being provided, **characterised in that** the means for determining a response characteristic comprise means (16, 17, 20) for making a zero test gas, to which the measurement gas sensor (5) and/or the correction gas sensor (51) is not sensitive, flow through the measurement chamber, and **in that** a control and evaluation unit (22) for controlling the means (16, 17, 20) for making a zero test gas flow through the measurement chamber is provided in order to initiate and terminate a flow of a zero test gas through the measurement chamber in a controlled manner, the control and evaluation unit (22) being designed to mathematically determine a response characteristic (32) from a time series (29) of measurement data recorded by means of the reading means (25) of the measurement gas sensor (5) and/or by means of the reading means (526) of the correction gas sensor (51), and/or being designed for mathematically compensating for a response characteristic (32) of the measurement data which are recorded by means of the reading means (5) of the measurement gas sensor (5).

**2.** Device (1) according to claim 1, **characterised in that** the means for determining a response characteristic comprise a correction gas sensor (51) that is sensitive to a correction gas and is intended for measuring a measurement variable characterising a partial pressure of the correction gas in the measurement chamber, and reading means (256) for reading the correction gas sensor (51), the correction gas being contained in the surrounding gas and/or being different from the measurement gas.

**3.** Device (1) according to either claim 1 or claim 2, **characterised in that** it comprises a zero test gas source (17) for providing the zero test gas, the zero test gas source (17) being designed to produce the zero test gas from the surrounding gas and/or comprising means for removing the measurement gas from the surrounding gas, and/or **in that** the zero test gas source (17) comprises means for chemically converting the measurement gas such that the measurement gas sensor (5) and/or the correction gas sensor (51) is not sensitive to gaseous conversion products that result from the conversion process.

**4.** Device (1) according to any of the preceding claims, **characterised in that**, upstream of the inlet opening (6) and/or downstream of the outlet opening (7), a membrane (4) that is permeable to the surrounding gas is arranged such that the surrounding gas enters the measurement chamber via the membrane (4) and/or the surrounding gas leaves the device via the membrane (4).

**5.** Device (1) according to any of the preceding claims, **characterised in that** a bypass line that connects the inlet opening (6) to the outlet opening (7) is provided.

**6.** Device (1) according to any of the preceding claims, **characterised in that** the measurement gas sensor (5) and/or the correction gas sensor (51) comprises an optical gas sensor, a non-dispersive infrared sensor and/or a spectrometer based on a tuneable laser diode and/or a sensor based on a tuneable laser diode, and/or **in that** a pump (9) is provided in order to circulate the surrounding gas through the measurement chamber.

**7.** Method for operating a device (1) comprising a measurement chamber and a measurement gas sensor (5) which is sensitive to a measurement gas contained in a surrounding gas and is intended for measuring a variable that characterises the partial pressure of the measurement gas in the measurement chamber, **characterised in that** it comprises a method for determining a response characteristic (32) of the device (1), in which:

a. a zero test gas, to which the measurement gas sensor (5) and/or a correction gas sensor (51), which is sensitive to a correction gas, is not sensitive, flows through the measurement chamber, an output signal of the measurement gas sensor (5) and/or an output signal of the correction gas sensor (51) being simultaneously monitored until it reaches a substantially constant minimum value;

b. a surrounding gas flows through the measurement chamber after the output signal has reached the minimum value, the output signal of the measurement gas sensor (5) and/or the output signal of the correction gas sensor (51) being recorded in a measurement series in a time-resolved manner during a calibration time interval (34) when the surrounding gas begins to flow through the measurement chamber;

c. the response characteristic is determined on the basis of an evaluation of the measurement series,

at least steps (a) and (b) being carried out *in situ.*

8. Method according to claim 7, **characterised in that** in order to evaluate the measurement series, a correction response characteristic is first determined on the basis of the output signal of the correction gas sensor (51) and the response characteristic of the device is subsequently determined from the correction response characteristic.

9. Method according to either claim 7 or claim 8, **characterised in that** the output signal of the measurement gas sensor (5) is corrected on the basis of the response characteristic in order to obtain a chronological assignment of the output signal to the partial pressure of the measurement gas at the time of recording the output signal.

10. Method according to any of claims 7 to 9, **characterised in that** the calibration time interval (34) is selected such that the partial pressure of the measurement gas and/or the partial pressure of the correction gas is substantially constant during the calibration time interval (34).

11. Method according to any of claims 7 to 10, **characterised in that**, for determining the response characteristic, a first portion (35) of the measurement series, which was recorded during a mixing phase of the surrounding gas with the zero test gas in the measurement chamber, is not considered, the first portion (35) of the measurement series extending as far as a transition point (37) from a substantially linear curve shape to a substantially exponential curve shape.

12. Method according to any of claims 7 to 11, **characterised in that** the response characteristic is determined on the basis of a regression analysis of the measurement series (29), the following model preferably being used:

$$p(t) = p_{water} + (p(t_0) - p_{water})e^{-\frac{t}{\tau}}$$

in which p(t) denotes the output signal at time t, $p_{water}$ denotes the partial pressure of the measurement gas within the surrounding gas, $p(t_0)$ denotes the output signal at time $t_0$ and $\tau$ denotes a time constant.

13. Method according to any of claims 7 to 12, **characterised in that** the output signal of the measurement gas sensor (5) is corrected on the basis of the response characteristic, by the output signal p(t) at time t being assigned to the partial pressure $p_{water}$ of the measurement gas within the surrounding gas at the time of recording the output signal, according to the following formula:

$$p_{water} = \frac{p(t) - p(t_0)e^{-\frac{t}{\tau}}}{1 - e^{-\frac{t}{\tau}}}$$

in which $p(t_0)$ denotes the output signal at time $t_0$.

14. Method according to any of claims 7 to 13, **characterised in that** the method for determining a response characteristic is carried out several times after each significant change in one or more ambient parameters.

**Revendications**

1. Dispositif (1) de détection d'une pression partielle d'un gaz de mesure contenu dans un gaz ambiant, comprenant un espace de mesure avec au moins une ouverture d'entrée (6) pour l'entrée de gaz ambiant dans l'espace de mesure et au moins une ouverture de sortie (7) pour la sortie de gaz ambiant de l'espace de mesure, un capteur de gaz de mesure (5) sensible au gaz de mesure pour la mesure d'une valeur de mesure caractéristique de la pression partielle du gaz de mesure dans l'espace de mesure ainsi que des moyens de lecture (25) pour la lecture du capteur de gaz de mesure (5), dans lequel des moyens de détermination d'une caractéristique de réponse (32) du dispositif (1) sont prévus, **caractérisé en ce que** les moyens de détermination d'une caractéristique de réponse comprennent des moyens (16, 17, 20) pour la traversée de l'espace de mesure par un gaz de mise à zéro, auquel le capteur de gaz de mesure (5) et/ou le capteur de gaz de correction (51) n'est pas sensible et **en ce qu'**une unité de commande et d'évaluation (22) est prévue pour la commande des moyens (16, 17, 20) pour la traversée de l'espace de mesure pour introduire et évacuer de manière ciblée une traversée de l'espace de mesure par un gaz de mise à zéro, dans lequel l'unité de commande et d'évaluation (22) est réalisée pour la détermination arithmétique d'une caractéristique de réponse (32) à partir d'une série temporelle (29) par le biais des moyens de lecture (25) du capteur de gaz de mesure (5) et/ou de données de mesure enregistrées par le biais des moyens de lecture (526) du capteur de gaz de correction (51) et/ou est configuré pour la compensation arithmétique d'une caractéristique de réponse (32) des données de mesure enregistrées par le biais des moyens de lecture (5) du capteur de gaz de mesure (5).

2. Dispositif (1) selon la revendication 1, **caractérisé en ce que** les moyens de détermination d'une caractéristique de réponse comprennent un capteur de gaz de correction (51) sensible à un gaz de correction pour la mesure d'une valeur de mesure caractéristique d'une pression partielle du gaz de correction dans l'espace de mesure ainsi que des moyens de lecture (256) pour la lecture du capteur de gaz de correction (51), dans lequel le gaz de correction est contenu dans le gaz ambiant et/ou est différent du gaz de mesure.

3. Dispositif (1) selon la revendication 1 ou 2, **caractérisé en ce qu'**il présente une source de gaz de mise à zéro (17) pour la mise à disposition du gaz de mise à zéro, dans lequel la source de gaz de mise à zéro (17) est réalisée pour la génération du gaz de mise à zéro à partir du gaz ambiant et/ou comprend des moyens pour l'élimination du gaz de mesure du gaz ambiant et/ou **en ce que** la source de gaz de mise à zéro (17) comprend des moyens pour la transformation chimique du gaz de mesure de manière que le capteur de gaz de mesure (5) et/ou le capteur de gaz de correction (51) n'est pas sensible à des produits de transformation gazeux générés lors de la transformation.

4. Dispositif (1) selon l'une quelconque des revendications précédentes, **caractérisé en ce qu'**une membrane perméable au gaz ambiant (4) est agencée en amont de l'ouverture d'entrée (6) et/ou en aval de l'ouverture de sortie (7) de telle manière que le gaz ambiant accède par la membrane (4) dans l'espace de mesure et/ou le gaz ambiant quitte le dispositif par la membrane (4).

5. Dispositif (1) selon l'une quelconque des revendications précédentes, **caractérisé en ce qu'**une conduite de dérivation reliant l'ouverture d'entrée (6) à l'ouverture de sortie (7) est prévue.

6. Dispositif (1) selon l'une quelconque des revendications précédentes, **caractérisé en ce que** le capteur de gaz de mesure (5) et/ou le capteur de gaz de correction (51) comprend un capteur de gaz optique, un capteur infrarouge non dispersif et/ou un spectromètre basé sur une diode laser accordable et/ou un capteur basé sur une diode laser accordable et/ou **en ce qu'**une pompe (9) est prévue pour faire circuler le gaz ambiant par l'espace de mesure.

7. Procédé de fonctionnement d'un dispositif (1) comprenant un espace de mesure et un capteur de gaz de mesure (5) sensible à un gaz de mesure contenu dans un gaz ambiant pour la mesure d'une valeur caractéristique de la pression partielle du gaz de mesure dans l'espace de mesure, **caractérisé en ce qu'**il comprend un procédé de détermination d'une caractéristique de réponse (32) du dispositif (1), dans lequel

    a. l'espace de mesure est traversé par un gaz de mise à zéro, auquel le capteur de gaz de mesure (5) et/ou un capteur de gaz de correction (51) sensible à un gaz de correction n'est pas sensible, dans lequel un signal de sortie du capteur de gaz de mesure (5) et/ou un signal de sortie du capteur de gaz de correction (51) est observé en même temps, jusqu'à ce que celui-ci atteigne une valeur minimum sensiblement constante ;

b. l'espace de mesure est traversé par un gaz ambiant, après que le signal de sortie a atteint la valeur minimum, dans lequel au début de la traversée de gaz ambiant, le signal de sortie du capteur de gaz de mesure (5) et/ou le signal de sortie du capteur de gaz de correction (51) est enregistré pendant un intervalle de temps d'étalonnage (34) avec une résolution temporelle dans une série de mesure ;

c. la caractéristique de réponse est déterminée à l'aide d'une évaluation de la série de mesure,

dans lequel au moins les étapes (a) et (b) sont réalisées *in situ*.

8. Procédé selon la revendication 7, **caractérisé en ce que** pour l'évaluation de la série de mesure, une caractéristique de réponse de correction est d'abord déterminée sur la base du signal de sortie du capteur de gaz de correction (51) et ensuite la caractéristique de réponse du dispositif est déterminée à partir de la caractéristique de réponse de correction.

9. Procédé selon la revendication 7 ou 8, **caractérisé en ce que** le signal de sortie du capteur de gaz de mesure (5) est corrigé à l'aide de la caractéristique de réponse pour obtenir une attribution temporelle du signal de sortie à la pression partielle du gaz de mesure au moment de la réception du signal de sortie.

10. Procédé selon l'une quelconque des revendications 7 à 9, **caractérisé en ce que** l'intervalle de temps d'étalonnage (34) est sélectionné de sorte que la pression partielle du gaz de mesure et/ou la pression partielle du gaz de correction soit sensiblement constante pendant l'intervalle de temps d'étalonnage (34).

11. Procédé selon l'une quelconque des revendications 7 à 10, **caractérisé en ce que** pour la détermination de la caractéristique de réponse, une première section (35) de la série de mesure, laquelle a été enregistrée pendant une phase de mélange du gaz ambiant avec le gaz de mise à zéro dans l'espace de mesure, n'est pas prise en compte, dans lequel la première section (35) de la série de mesure s'étend jusqu'à un point de transition (37) d'une allure de courbe sensiblement linéaire à une allure de courbe sensiblement exponentielle.

12. Procédé selon l'une quelconque des revendications 7 à 11, **caractérisé en ce que** la caractéristique de réponse est déterminée à l'aide d'une analyse de régression de la série de mesure (29), dans lequel de préférence le modèle suivant est appliqué :

$$p_{(t)} = p_{water} + \left(p_{(t_0)} - p_{water}\right)e^{-\frac{t}{\tau}},$$

dans lequel p(t) désigne le signal de sortie au moment t, $p_{water}$ la pression partielle du gaz de mesure à l'intérieur du gaz ambiant, $p(t_0)$ le signal de sortie au moment $t_0$ et $\tau$ une constante de temps.

13. Procédé selon l'une quelconque des revendications 7 à 12, **caractérisé en ce que** le signal de sortie du capteur de gaz de mesure (5) est corrigé à l'aide de la caractéristique de réponse en attribuant le signal de sortie p(t) au moment t à la pression partielle $p_{water}$ du gaz de mesure à l'intérieur du gaz ambiant au moment de la réception du signal de sortie conformément à la formule suivante :

$$p_{water} = \frac{p_{(t)} - p_{(t_0)}e^{-\frac{t}{\tau}}}{1 - e^{-\frac{t}{\tau}}},$$

dans lequel $p(t_0)$ désigne le signal de sortie au moment $t_0$.

14. Procédé selon l'une quelconque des revendications 7 à 13, **caractérisé en ce que** le procédé de détermination d'une caractéristique de réponse est réalisé plusieurs fois, après chaque modification significative d'un ou plusieurs paramètres ambiants.

Fig.1

# Fig.2

# Fig.3

# Fig.4

**EP 2 629 082 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006019705 B3
- WO 9840722 A1
- DE 102006035788 A1